# EUROPEAN PATENT SPECIFICATION

(11) **EP 3 431 036 B1**
(45) Date of publication and mention of the grant of the patent: **07.09.2022**
(21) Application number: 18382511.6
(22) Date of filing: 09.07.2018
(51) Int. Cl.: A61D 9/00, A61F 5/058

(54) **BRACE FOR A FRACTURED LEG OF A QUADRUPED ANIMAL**
BESCHLAG FÜR EIN GEBROCHENES BEIN EINES VIERBEINIGEN TIERES
ATTELLE POUR PIED FRACTURÉ D'UN ANIMAL QUADRUPÈDE

(30) Priority: 18.07.2017 ES 201730945
(43) Date of publication of application: 23.01.2019
(73) Proprietor: Ostolaza Salegi, Juan Jose, 20850 Mendaro (ES)
(72) Inventor: OSTOLAZA SALEGI, Juan Jose, 20850 MENDARO (ES); ISUSQUIZA GARCIA, Erik, 20720 AZKOITIA (ES); LOZARES ABASOLO, Jokin, 20500 ARRASATE - MONDRAGON (ES)
(74) Representative: Igartua, Ismael

(56) References cited:
- CN-U- 202 843 868
- US-A- 4 029 090
- US-A1- 2016 346 070

## Description

### TECHNICAL FIELD

The present invention relates to braces for legs of quadruped animals.

### PRIOR ART

Braces configured for holding therein the injured limb of a quadruped animal, such as a leg, such that the animal rests on said brace so as to not exacerbate the injury of the animal, are known.

In this sense, patent document US4029090 A1 discloses a brace for a foreleg of a quadruped animal such as a horse. The brace is cylindrical and holds therein the injured leg of the animal. The animal rests on the brace and not on the injured leg. The brace is fastened to a harness covering the back of the animal by fastening means.

CN202843868 U describes a brace to treat fractures of human legs. The brace comprises a leg fixing bracket, a knee fixing bracket and a foot fixing bracket. The knee fixing bracket is fixedly arranged above the leg fixing bracket and the foot fixing bracket is arranged below the leg fixing bracket, being connected to the leg fixing bracket in a sliding mode.

### DISCLOSURE OF THE INVENTION

The object of the invention is to provide a brace for an injured leg of a quadruped animal, as defined in the claims.

The brace according to the invention is adapted for a fractured leg of a quadruped animal such as a cow or a horse, and comprises a support structure configured for housing therein the injured leg of the animal, preventing said leg from resting on the ground. the brace comprises an immobilizing structure configured for immobilizing the injured leg by pressure, said immobilizing structure being attached to the support structure in such a way that the immobilizing structure can be slide with respect to the support structure.

The support structure comprises an upper support configured for being supported on the lower part of the chest or of the thigh of the animal, and a lower base, and main columns arranged between the upper support and the lower base, such that the animal rests on the support structure being supported on the lower base.

The immobilizing structure can be slide guided on the main columns of the support structure.

The fractured leg of the quadruped animal can heal properly with the brace of the invention as it enables the two fractured ends of the bone to be arranged close to one another and immobilized in said position so that the bone can weld properly while the brace of the invention serves, at the same time, as a crutch to prevent the animal from resting its weight on the fractured or injured area, which thereby prevents exacerbating the injury of the animal.

These and other advantages and features of the invention will become evident in view of the drawings and detailed description of the invention.

### DESCRIPTION OF THE DRAWINGS

Figure 1 shows a perspective view of the brace according to an embodiment of the invention.
Figure 2A shows a perspective view of the support structure of the brace of Figure 1.
Figure 2B shows a front view of the support structure of the brace of Figure 1.
Figure 3A shows a perspective view of the immobilizing structure of the brace of Figure 1.
Figure 3B shows a front view of the immobilizing structure of the brace of Figure 1.
Figure 4 shows a perspective view of the brace according to another embodiment of the invention.
Figure 5A shows a perspective view of the support structure of the brace of Figure 4.
Figure 5B shows a front view of the support structure of the brace of Figure 4.
Figure 6 shows a perspective view of the immobilizing structure of the brace of Figure 4.

### DETAILED DISCLOSURE OF THE INVENTION

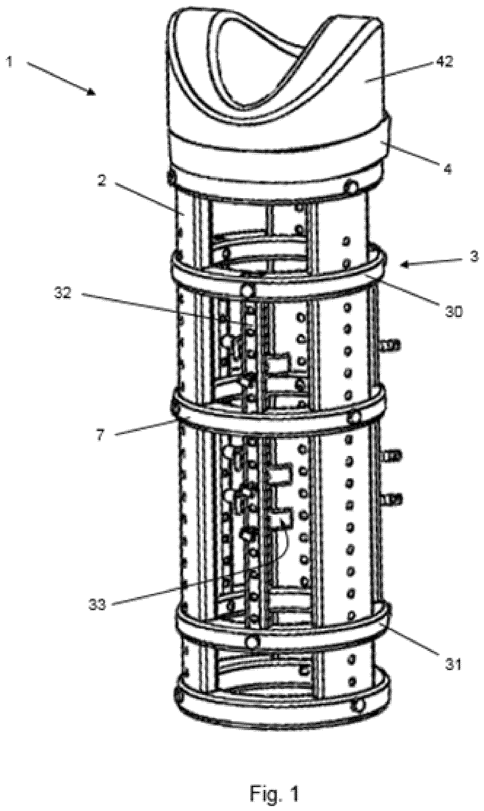

Figure 1 shows a brace 1 for a fractured or injured leg of a quadruped animal such as a cow or a horse according to an embodiment of the invention, comprising a support structure 2 configured for housing therein the injured leg of the animal, preventing the animal from resting on the fractured area. The brace 1 also comprises an immobilizing structure 3 configured for immobilizing the injured leg by pressure, the immobilizing structure 3 being attached to the support structure 2.

The injured leg of the quadruped animal can heal properly with the brace 1 of the invention as it enables the two fractured ends of the bone to be arranged close to one another and immobilized in said position so that the bone can weld properly while the brace 1 serves, at the same time, as a crutch to prevent the animal from resting its weight on the fractured or injured area, thereby favoring proper healing of the injury of the animal.

Although reference has been made throughout the description to a fractured leg, the brace 1 of the invention is also suitable to help heal any type of injury such as a sprain or an open wound, for example.

According to the invention, the immobilizing structure 3 is slidable with respect to the support structure 2, such that transmission of impacts or vibrations absorbed by the support structure 2 to the injured leg is prevented since the immobilizing structure 3 dampens said impacts, therefore, the injured leg of the animal does not have to endure uneven terrains.

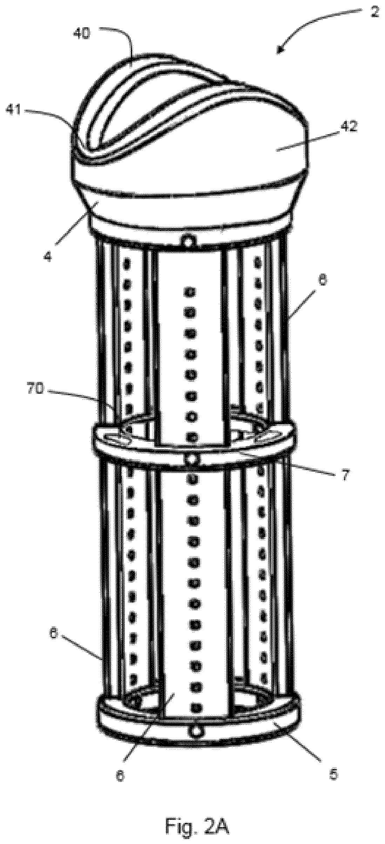

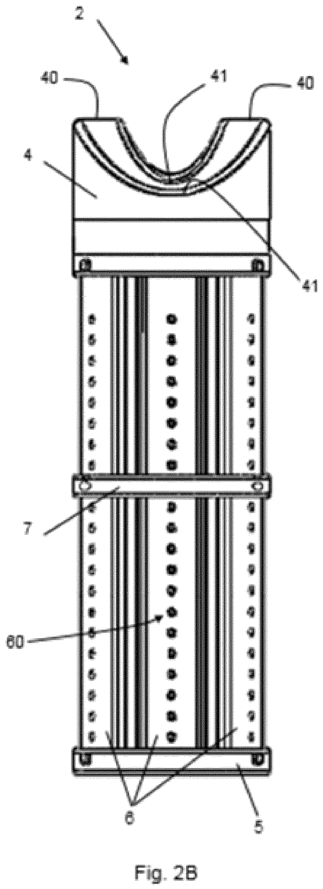

According to the invention, the support structure 2 comprises an upper support 4, shown in Figures 1, 2A, and 2B, configured for being supported on the lower part of the chest, if it is used on a foreleg, or on the lower part of the thigh, if it is used on a hind leg. To prevent said upper support 4 from chafing the animal, it comprises a contact area, with an anatomical shape, comprising at least one layer of foamy material to prevent chafing while at the same time providing a soft support area.

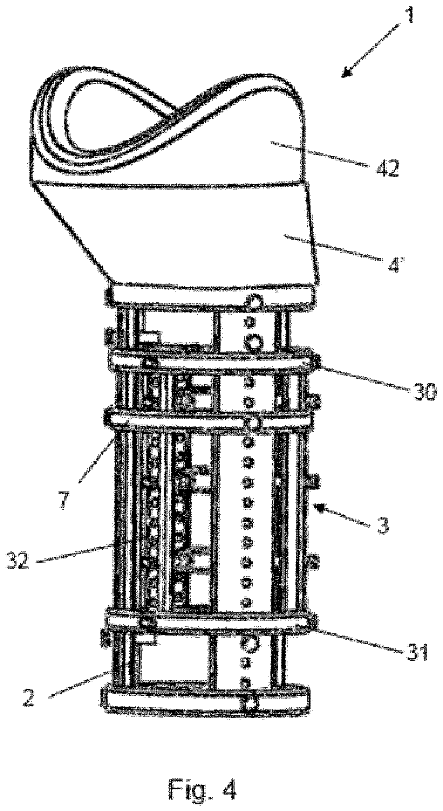

The example of Figures 1, 2A, 2B, 3A, and 3B shows a brace 1 for a foreleg. The brace 1 for a hind leg is very similar to the brace of a foreleg, the latter, i.e. the brace 1 for a hind leg, being configured for housing therein the hock, therefore, the diameter of the brace 1 for a hind leg will be somewhat larger, as shown in Figures 4, 5A, 5B, and 6. In this case, the upper support 4' of the brace 1 for the hind leg is also somewhat larger since it is configured for being supported on the lower part of the thigh.

The anatomy contact area 42 according to the invention, the contact area being understood as the upper area of the upper support 4 or 4', is symmetrical such that it is capable of adapting to the contour of the lower part of the chest or thigh of the animal regardless of whether the brace is placed on a right or left leg.

According to the invention, each half of the anatomy contact area 42 comprises a curved ridge 40 leading at each end to a curved valley 41 joining both ridges 40, as seen in Figures 2A, 2B, 4, and 5B. The lower part of the chest or thigh of the animal is preferably supported on said valleys 41. Although the shape of the described contact area is the preferred one, other anatomical contact shapes are not dismissed.

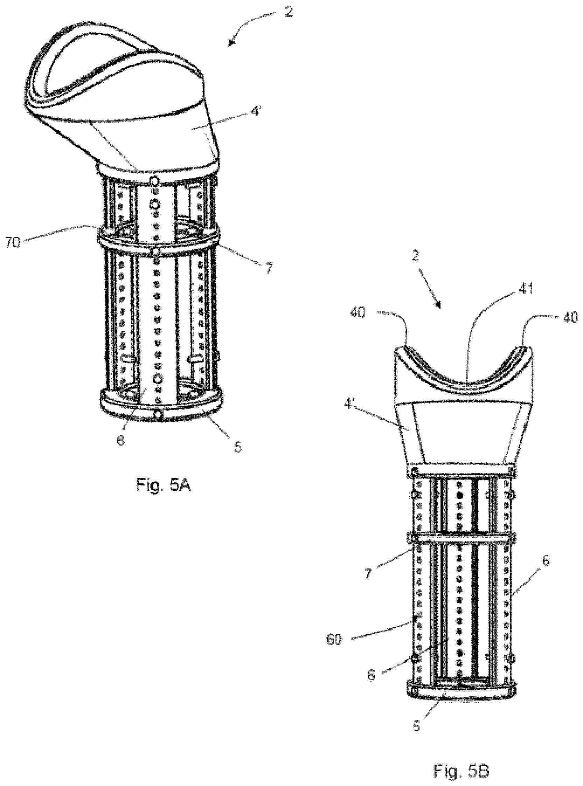

The support structure 2 of the invention, shown in Figures 2A and 2B for a foreleg or in Figures 5A and 5B for a hind leg, also comprises a lower base 5 which is attached to the upper support 4 and 4' through main columns 6 which are arranged between the upper support 4 and 4' and said lower base 5, such that the animal rests on the support structure 2 being supported on the lower base 5. As seen in the drawings, the brace 1 according to the preferred embodiment of the invention comprises three main columns 6 arranged at 120 degrees.

The lower base 5 and the upper support according to any of the embodiments 4 or 4' are circular, providing the support structure 2 with a circular configuration which is capable of housing therein the injured leg of the animal.

The immobilizing structure 3 is slidable with respect to the support structure 2, sliding and being guided on the main columns 6 of the support structure 2.

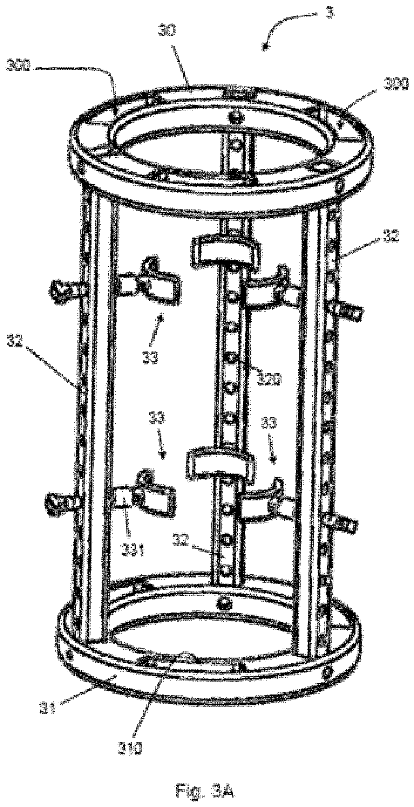

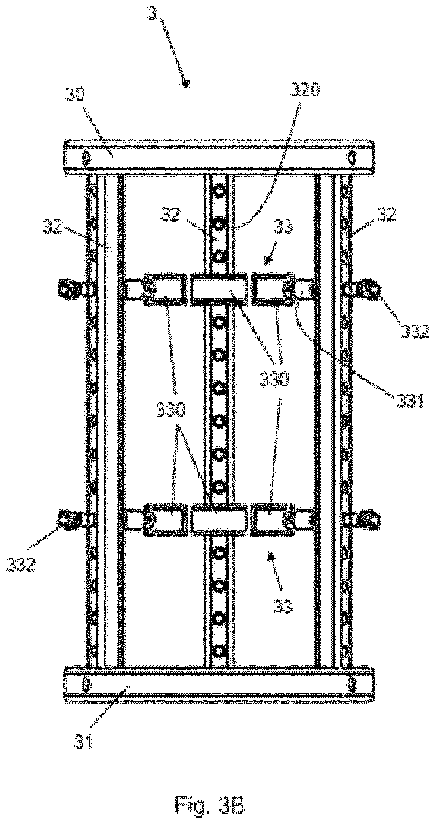

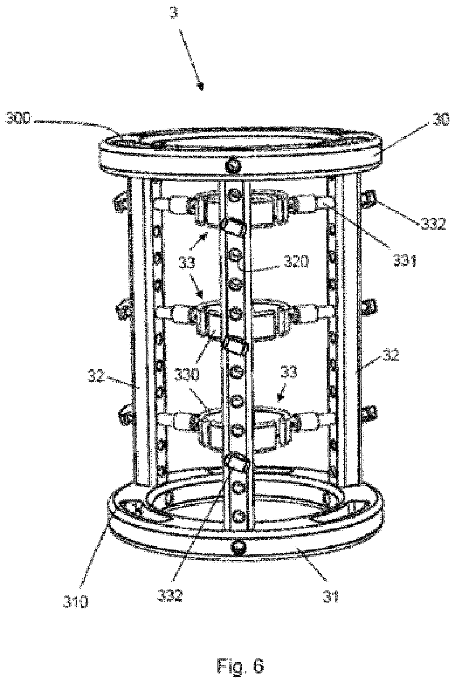

As seen in Figures 3A, 3B, and 6, the immobilizing structure 3 according to the preferred embodiment of the invention comprises an upper ring 30 and a lower ring 31 attached by means of fixing columns 32 such that the main columns 6 of the support structure 2 go through the upper ring 30 and the lower ring 31, the immobilizing structure 3 being arranged between the upper support 4 or 4' and the lower base 5 of the support structure 2, as seen in Figure 1 or in Figure 4. The immobilizing structure 3 can be slid on the support structure 2, guided on said main columns 6. To that end, the upper ring 30 and the lower ring 31 comprise passage means which allow the passage of the corresponding main columns 6, said passage means comprising a hole 300 per each main column 6, preferably rectangular, although it may comprise any shape that allows the passage of the corresponding main column 6.

Therefore, as mentioned above, the immobilizing structure 3 can absorb the impacts or vibrations to which the support structure 2 may be subjected, such that transmission of these impacts or vibrations to the injured leg of the animal is prevented.

According to the preferred embodiment of the invention, the immobilizing structure 3 comprises three fixing columns 32 arranged at 120° for a foreleg, as seen in Figures 3A or 3B, or for a hind leg, in Figure 6.

Likewise, the immobilizing structure 3 comprises a plurality of immobilizing elements 33 configured for immobilizing the injured leg of the animal by pressure, i.e., said immobilizing elements 33 are supported against the leg of the animal and fixed in said position, as will be described below.

In the non-limiting example of Figures 3A, 3B, and 6, each fixing column 32 comprises two or three immobilizing elements 33 arranged at different heights (although the possibility of using more immobilizing elements 33 is not ruled out), such that said plurality of immobilizing elements 33 surrounds and secures the leg of the animal, immobilizing it, so that it can heal, preventing the animal from resting its weight on the injured area, thereby favoring the proper healing of the injury. Likewise, since the animal rests on the support structure 2, and not on the injured area of the leg, the animal can walk without exacerbating the injury of the leg.

Since the immobilizing elements 33 are removable, they can be arranged close to the fractured or injured area such that it is possible to enclose the area that is the closest possible to the injury, without pressing on the affected area, and immobilizing said area so that the injury can heal effectively.

To that end, each fixing column 32 comprises coupling means 320 which allow removably fixing the immobilizing elements 330 at different heights. In the non-limiting example of the invention, said coupling means 320 comprise through holes 320 arranged aligned along each of the corresponding columns 32, as seen in Figures 3A, 3B, and 6.

According to the preferred embodiment of the invention, each immobilizing element 33 comprises a half-moon-shaped contact end 330 configured for being supported on the injured leg of the animal. Said contact end 330 is attached to an end of a rod 331 going through the coupling means 320, i.e., one of the through holes 320 of the corresponding fixing column 32. The immobilizing element 33 is fixed at the opposite end through abutment means, for example, a nut 332, which allow fixing the immobilizing element 33 between the leg of the animal and the corresponding fixing column 32. Said nut 332 cooperates with the threaded free end of the rod 331 projecting towards the outside of the brace 1.

To prevent chafing and to provide a soft support, the contact end can be lined with a foamy material like the contact area 42 of the upper support 4 of the support structure 2.

To strengthen the brace 1 of the invention, the support structure 2 comprises an intermediate ring 7 arranged between the upper support 4 or 4' and the lower base 5, the main columns 6 going through said intermediate ring 7, as seen in Figures 1 and 4. Said intermediate ring 7 is arranged in a movable manner on the support structure 2, i.e., the position thereof between the upper support 4 or 4' and the lower base 5 can be fixed at different heights if necessary. This is advantageous in the event that the injury coincides with the intermediate ring 7. In this case, i.e., if the injury and the position of the intermediate ring 7 coincide, said ring 7 can be moved upwards or downwards to enable proper immobilization of the affected area.

The intermediate ring 7 comprises passage means which allow the passage of the corresponding main columns 6, said passage means comprising a hole for every main column 6, preferably rectangular, although it may comprise any shape that allows the passage of the corresponding main column 6. In turn, each main column 6 comprises coupling means 60 which allow removably fixing the intermediate ring 7 at different heights. In the non-limiting example of the invention, said coupling means 60 comprise through holes 60 arranged aligned along each of the main columns 6, as seen in Figures 2A, 2B, 5A, and 5B. The intermediate ring 7 is fixed to the main columns 6 at the desired height through attachment means, for example, screws, which go through the coupling means, i.e., a corresponding hole 60, to be fixed through the opposite end preferably with a corresponding nut.

Likewise, the fixing columns 32 of the immobilizing structure 3 go through the intermediate ring 7, as seen in Figure 1 or 4, such that said intermediate ring 7 is arranged between the upper ring 30 and the lower ring 31 of the immobilizing structure 3. The fixing columns 32 of the immobilizing structure 3 are thereby strengthened by said intermediate ring 7.

To that end, according to the preferred embodiment of the invention, the intermediate ring 7 comprises passage openings 70 which allow the passage of the fixing columns 32 of the immobilizing structure 3. Each passage opening 70 is preferably arranged between two main columns 6 of the support structure 2, as shown in Figure 1.

Optionally, the main columns 6 of the support structure 2 can be telescopic, which allows adapting the brace 1 of the invention to quadruped animals of different heights in a simple manner.

In the preferred embodiment of the invention, both the lower base 5 and the upper support 4 or 4' of the support structure 2 are removably attached to the main columns 6. The upper ring 30 and the lower ring 31 of the immobilizing structure 3 are also removably attached to the fixing columns 32. This allows easily assembling and disassembling the brace 1, for example, for replacing any of the components in the event of wear.

The brace 1 of the invention is assembled according to a preferred method, although it is not the only method of assembly.

According to said preferred method of assembly, in a first step the main columns 6 of the support structure 2 are fixed to the upper support 4 or 4'. The immobilizing structure 3 can be assembled at the same time. To that end, the fixing columns 32 are fixed to the upper ring 30, then the intermediate ring 7 of the support structure 2 is placed on the fixing columns 32 of the immobilizing structure 3, and finally the lower ring 31 is placed.

Once the immobilizing structure 3 having the intermediate ring 7 incorporated therein has been assembled, it is assembled on the main columns 6 of the support structure 2, and the lower base 5 is then placed and fixed on the main columns 6. Finally, the intermediate ring 7 is fixed to the main columns 6 at the desired height, the immobilizing structure 3 being arranged such that it can be slid on the main columns 6 of the support structure 2.

The immobilizing elements 33 can be assembled anytime, for example, when the immobilizing structure 3 is being assembled or at the end of the assembly of the brace 1.

The fractured or injured leg of the quadruped animal is inserted into the brace 1 already assembled for use, and the immobilizing elements 33 are then fixed, immobilizing the injured area. As a result of the immobilizing elements 33, the immobilizing structure 3 is fixed with respect to the leg of the animal. When the animal is returned to a standing position, the support structure 2, the length of which is slightly greater than the affected leg, acts as a crutch, preventing the animal from resting its weight on the fractured or injured area, which prevents exacerbating the injury of the animal.

## Claims

1. Brace for a fractured leg of a quadruped animal such as a cow or a horse, comprising a support structure (2) configured for housing therein the fractured leg, preventing said leg from resting on the ground, the brace (1) comprising an immobilizing structure (3) configured for immobilizing the fractured leg by pressure, the immobilizing structure (3) being attached to the support structure (2), wherein the immobilizing structure (3) is slidable with respect to the support structure (2), **characterized in that** the support structure (2) comprises an upper support (4; 4') configured for being supported on the lower part of the chest or of the thigh of the animal, a lower base (5), and main columns (6) which are arranged between the upper support (4; 4') and the lower base (5), such that the animal rests on the support structure (2) being supported on the lower base (5), where the immobilizing structure (3) can be slid being guided on the main columns (6) of the support structure (2).

2. Brace according to claim 1, wherein the support structure (2) comprises three main columns (6) arranged at 120°.

3. Brace according to claim 1 or 2, wherein the immobilizing structure (3) comprises an upper ring (30) and a lower ring (31) attached by means of fixing columns (32), preferably three arranged at 120°, such that the main columns (6) of the support structure (2) go through the upper ring (30) and the lower ring (31), the immobilizing structure (3) being arranged between the upper support (4; 4') and the lower base (5) of the support structure (2).

4. Brace according to claim 3, wherein the immobilizing structure (3) comprises a plurality of immobilizing elements (33) suitable for immobilizing the injured leg of the animal by pressure, each immobilizing element (33) comprising a half-moon-shaped contact end (330) which rests against the injured leg of the animal.

5. Brace according to claim 4, wherein each fixing column (32) comprises coupling means (320), preferably through holes (320) arranged aligned along the corresponding column (32), which allow removably fixing the immobilizing elements (33) at different heights.

6. Brace according to claim 5, wherein each immobilizing element (33) comprises a rod (331) attached at an end to the half-moon-shaped contact end (330), said rod (331) going through the corresponding coupling means (320) to be fixed against the corresponding fixing column (32) at the opposite end through abutment means, said abutment means preferably comprising a nut (332) cooperating with the threaded free end of a corresponding rod (331).

7. Brace according to any of claims 3 to 6, wherein the support structure (2) comprises an intermediate ring (7) arranged in a movable manner between the upper support (4) and the lower base (5) such that the main columns (6) go through said intermediate ring (7).

8. Brace according to claim 7, wherein the intermediate ring (7) comprises passage means to allow the passage of the corresponding main columns (6), said passage means preferably comprising a hole for every main column (6).

9. Brace according to claim 7 or 8, wherein each main column (6) comprises coupling means, preferably through holes (60) arranged aligned along the corresponding main column (6), which allow removably fixing the intermediate ring (7) to the support structure (2) at different heights.

10. Brace according to any of claims 7 to 9, wherein the fixing columns (32) of the immobilizing structure (3) go through the intermediate ring (7) such that said intermediate ring (7) is arranged between the upper ring (30) and the lower ring (31) of the immobilizing structure (3).

11. Brace according to claim 10, wherein the intermediate ring (7) comprises passage openings (70) which allow the passage of the fixing columns (32) of the immobilizing structure (3), a passage opening (70) preferably being arranged between two main columns (6) of the support structure (2).

12. Brace according to any of the preceding claims, wherein the main columns (6) of the support structure (2) are telescopic.

13. Brace according to any of the preceding claims, wherein the upper support (4; 4') comprises an anatomy contact area (42) which is capable of adapting to the contour of the lower part of the chest or thigh of the animal regardless of whether the brace (1) is placed on a right or left leg, said anatomy contact area preferably comprising at least one layer of foamy material.

14. Brace according to claim 13, wherein the anatomy contact area (42) is symmetrical, each half comprising a curved ridge (40) leading at each end to a curved valley (41) joining both ridges (40).

## Patentansprüche

1. Schiene für ein gebrochenes Bein eines vierbeinigen Tieres, wie einer Kuh oder eines Pferdes, umfassend eine Stützstruktur (2), die ausgebildet ist, um das gebrochene Bein darin aufzunehmen, wobei verhindert wird, dass das Bein auf dem Boden ruht, wobei die Schiene (1) eine Immobilisierungsstruktur (3) umfasst, die ausgebildet ist, um das gebrochene Bein durch Druck zu immobilisieren, wobei die Immobilisierungsstruktur (3) an der Stützstruktur (2) befestigt ist, wobei die Immobilisierungsstruktur (3) in Bezug auf die Stützstruktur (2) verschiebbar ist, **dadurch gekennzeichnet, dass** die Stützstruktur (2) eine obere Stütze (4; 4'), die so ausgebildet ist, dass sie auf dem unteren Teil des Brustkorbs oder des Oberschenkels des Tieres abgestützt wird, eine untere Basis (5) und Hauptsäulen (6), die zwischen der oberen Stütze (4; 4') und der unteren Basis (5) angeordnet sind, umfasst, so dass das Tier auf der Stützstruktur (2) ruht, die auf der unteren Basis (5) abgestützt ist, wobei die Immobilisierungsstruktur (3) gleitend an den Hauptsäulen (6) der Stützstruktur (2) geführt werden kann.

2. Schiene gemäß Anspruch 1, wobei die Stützstruktur (2) drei Hauptsäulen (6) umfasst, die in einem Winkel von 120° angeordnet sind.

3. Schiene gemäß Anspruch 1 oder 2, wobei die Immobilisierungsstruktur (3) einen oberen Ring (30) und einen unteren Ring (31) umfasst, die mittels Befestigungssäulen (32) befestigt sind, vorzugsweise drei, die unter 120° angeordnet sind, so dass die Hauptsäulen (6) der Stützstruktur (2) durch den oberen Ring (30) und den unteren Ring (31) verlaufen, wobei die Immobilisierungsstruktur (3) zwischen der oberen Stütze (4; 4') und der unteren Basis (5) der Stützstruktur (2) angeordnet ist.

4. Schiene gemäß Anspruch 3, wobei die Immobilisierungsstruktur (3) eine Vielzahl von immobilisierenden Elementen (33) umfasst, die geeignet sind, das verletzte Bein des Tieres durch Druck zu immobilisieren, wobei jedes immobilisierende Element (33) ein halbmondförmiges Kontaktende (330) umfasst, das an dem verletzten Bein des Tieres anliegt.

5. Schiene gemäß Anspruch 4, wobei jede Befestigungssäule (32) Kopplungsmittel (320) umfasst, vorzugsweise Durchgangslöcher (320), die entlang der entsprechenden Säule (32) ausgerichtet sind, die es ermöglichen, die immobilisierende Elemente (33) auf verschiedenen Höhen lösbar zu befestigen.

6. Schiene gemäß Anspruch 5, wobei jedes immobilisierendes Element (33) eine Stange (331) umfasst, die an einem Ende an dem halbmondförmigen Kontaktende (330) befestigt ist, wobei die Stange (331) durch die entsprechenden Kopplungsmittel (320) hindurchgeht, um an der entsprechenden Befestigungssäule (32) an dem gegenüberliegenden Ende durch Anschlagsmittel befestigt zu werden, wobei die Anschlagsmittel vorzugsweise eine Mutter (332) umfassen, die mit dem freien Gewindeende einer entsprechenden Stange (331) zusammenwirkt.

7. Schiene gemäß einem der Ansprüche 3 bis 6, wobei die Stützstruktur (2) einen Zwischenring (7) umfasst, der beweglich zwischen der oberen Stütze (4) und der unteren Basis (5) angeordnet ist, so dass die Hauptsäulen (6) durch den Zwischenring (7) hindurchgehen.

8. Schiene gemäß Anspruch 7, wobei der Zwischenring (7) Durchgangsmittel umfasst, um den Durchgang der entsprechenden Hauptsäulen (6) zu ermöglichen, wobei die Durchgangsmittel vorzugsweise ein Loch für jede Hauptsäule (6) umfassen.

9. Schiene gemäß Anspruch 7 oder 8, wobei jede Hauptsäule (6) Kopplungsmittel umfasst, vorzugsweise Durchgangslöcher (60), die entlang der entsprechenden Hauptsäule (6) ausgerichtet sind, die es ermöglichen, den Zwischenring (7) an der Stützstruktur (2) auf verschiedenen Höhen lösbar zu befestigen.

10. Schiene gemäß einem der Ansprüche 7 bis 9, wobei die Befestigungssäulen (32) der Immobilisierungsstruktur (3) durch den Zwischenring (7) gehen, so dass der Zwischenring (7) zwischen dem oberen Ring (30) und dem unteren Ring (31) der Immobilisierungsstruktur (3) angeordnet ist.

11. Schiene gemäß Anspruch 10, wobei der Zwischenring (7) Durchgangsöffnungen (70) umfasst, die den Durchgang der Befestigungssäulen (32) der Immobilisierungsstruktur (3) ermöglichen, wobei eine Durchgangsöffnung (70) vorzugsweise zwischen zwei Hauptsäulen (6) der Stützstruktur (2) angeordnet ist.

12. Schiene gemäß einem der vorhergehenden Ansprüche, wobei die Hauptsäulen (6) der Stützstruktur (2) teleskopierbar sind.

13. Schiene gemäß einem der vorhergehenden Ansprüche, wobei die obere Stütze (4; 4') einen Anatomie-Kontaktbereich (42) umfasst, der fähig ist, sich an die Kontur des unteren Teils der Brust oder des Oberschenkels des Tieres anzupassen, unabhängig davon, ob die Schiene (1) an einem rechten oder linken Bein platziert ist, wobei der Anatomie-Kontaktbereich vorzugsweise mindestens eine Schicht aus Schaummaterial umfasst.

14. Schiene gemäß Anspruch 13, wobei der Anatomie-Kontaktbereich (42) symmetrisch ist, wobei jede Hälfte einen gekrümmten Steg (40) umfasst, der an jedem Ende zu einem gekrümmten Tal (41) führt, das beide Stege (40) verbindet.

## Revendications

1. Attelle pour pied fracturé d'un animal quadrupède tel qu'une vache ou un cheval, comprenant une structure de support (2) configurée pour y loger le pied fracturé, empêchant ledit pied de reposer sur le sol, l'attelle (1) comprenant une structure d'immobilisation (3) configurée pour immobiliser le pied fracturé par pression, la structure d'immobilisation (3) étant fixée à la structure de support (2), dans laquelle la structure d'immobilisation (3) peut coulisser par rapport à la structure de support (2), **caractérisée en ce que** la structure de support (2) comprend un support supérieur (4 ; 4') configuré pour être supporté sur la partie inférieure de la poitrine ou de la cuisse de l'animal, une base inférieure (5), et des colonnes principales (6) qui sont disposées entre le support supérieur (4 ; 4') et la base inférieure (5), de sorte que l'animal repose sur la structure de support (2) en étant supporté sur la base inférieure (5), où la structure d'immobilisation (3) peut être glissée en étant guidée sur les colonnes principales (6) de la structure de support (2).

2. Attelle selon la revendication 1, dans laquelle la structure de support (2) comprend trois colonnes principales (6) disposées à 120°.

3. Attelle selon la revendication 1 ou 2, dans laquelle la structure d'immobilisation (3) comprend une bague supérieure (30) et une bague inférieure (31) attachées au moyen de colonnes de fixation (32), de préférence trois disposées à 120°, de sorte que les colonnes principales (6) de la structure de support (2) traversent la bague supérieure (30) et la bague inférieure (31), la structure d'immobilisation (3) étant disposée entre le support supérieur (4 ; 4') et la base inférieure (5) de la structure de support (2).

4. Attelle selon la revendication 3, dans laquelle la structure d'immobilisation (3) comprend une pluralité d'éléments d'immobilisation (33) adaptés pour immobiliser par pression le pied blessé de l'animal, chaque élément d'immobilisation (33) comprenant une extrémité de contact (330) en forme de demi-lune qui repose contre le pied blessé de l'animal.

5. Attelle selon la revendication 4, dans laquelle chaque colonne de fixation (32) comprend des moyens d'accouplement (320), de préférence des trous traversants (320) disposés en alignement le long de la colonne correspondante (32), qui permettent de fixer de manière amovible les éléments d'immobilisation (33) à différentes hauteurs.

6. Attelle selon la revendication 5, dans laquelle chaque élément d'immobilisation (33) comprend une tige (331) fixée à une extrémité à l'extrémité de contact en forme de demi-lune (330), ladite tige (331) traversant les moyens de couplage (320) correspondants pour être fixée contre la colonne de fixation (32) correspondante à l'extrémité opposée par des moyens de butée, lesdits moyens de butée comprenant de préférence un écrou (332) coopérant avec l'extrémité libre filetée d'une tige (331) correspondante.

7. Contrefort selon l'une quelconque des revendications 3 à 6, dans lequel la structure de support (2) comprend un anneau intermédiaire (7) disposé de manière mobile entre le support supérieur (4) et la base inférieure (5) de telle sorte que les colonnes principales (6) traversent ledit anneau intermédiaire (7).

8. Attelle selon la revendication 7, dans laquelle la bague intermédiaire (7) comprend des moyens de passage pour permettre le passage des colonnes principales (6) correspondantes, lesdits moyens de passage comprenant de préférence un trou pour chaque colonne principale (6).

9. Attelle selon la revendication 7 ou 8, dans laquelle chaque colonne principale (6) comprend des moyens de couplage, de préférence des trous traversants (60) disposés en alignement le long de la colonne principale (6) correspondante, qui permettent de fixer de manière amovible l'anneau intermédiaire (7) à la structure de support (2) à différentes hauteurs.

10. Attelle selon l'une quelconque des revendications 7 à 9, dans laquelle les colonnes de fixation (32) de la structure d'immobilisation (3) traversent l'anneau intermédiaire (7) de sorte que ledit anneau intermédiaire (7) est disposé entre l'anneau supérieur (30) et l'anneau inférieur (31) de la structure d'immobilisation (3).

11. Attelle selon la revendication 10, dans laquelle l'anneau intermédiaire (7) comprend des ouvertures de passage (70) qui permettent le passage des colonnes de fixation (32) de la structure d'immobilisation (3), une ouverture de passage (70) étant de préférence disposée entre deux colonnes principales (6) de la structure de support (2).

12. Attelle selon l'une quelconque des revendications précédentes, dans laquelle les colonnes principales (6) de la structure de support (2) sont télescopiques.

13. Attelle selon l'une quelconque des revendications précédentes, dans laquelle le support supérieur (4 ; 4') comprend une zone de contact anatomique (42) apte à s'adapter au contour de la partie inférieure du thorax ou de la cuisse de l'animal, que l'attelle (1) soit placée sur une jambe droite ou gauche, ladite zone de contact anatomique comprenant de préférence au moins une couche de matériau mousseux.

14. Attelle selon la revendication 13, dans laquelle la zone de contact anatomique (42) est symétrique, chaque moitié comprenant une crête incurvée (40) menant à chaque extrémité à une vallée incurvée (41) reliant les deux crêtes (40).
